Europäisches Patentamt

European Patent Office

Office européen des brevets

(11) Publication number: **0 362 438**
**A1**

# EUROPEAN PATENT APPLICATION

(21) Application number: **88201279.2**

(22) Date of filing: **21.06.88**

(51) Int. Cl.⁵: **A01N 1/02**

(43) Date of publication of application:
**11.04.90 Bulletin 90/15**

(84) Designated Contracting States:
**DE**

(71) Applicant: **Boon, Mathilde Elisabeth**
**Achter de Hor 2**
**NL-9304 TA Lieveren(NL)**

Applicant: **Kok, Lanbrecht Piet**
**Achter de Hor 2**
**NL-9304 TA Lieveren(NL)**

Applicant: **Visser, Cornelis Elise**
**Graaf Willem de Oudelaan 38**
**NL-1412 AV Naarden(NL)**

(72) Inventor: **Boon, Mathilde Elisabeth**
**Achter de Hor 2**
**NL-9304 TA Lieveren(NL)**
Inventor: **Kok, Lanbrecht Piet**
**Achter de Hor 2**
**NL-9304 TA Lieveren(NL)**
Inventor: **Visser, Cornelis Elise**
**Graaf Willem de Oudelaan 38**
**NL-1412 AV Naarden(NL)**

(54) Microwave treatment of xenogeneic cartilage transplants.

(57) A method of preservation of bone, especially human cartilage, in which a bone structure is exposed to microwave irradiation.

EP 0 362 438 A1

## Introduction

Resorption of allogeneic cartilage implants is one of the problems in corrective facial and nasal surgery by which on the long-term a initially good operation result may become poor. However, the use of preserved costal cartilage has still proven to be indispensible.

Autogeneic cartilage has the advantage of toleration without immunological reaction of the host tissue, but even then signs of resorption can sometimes be noticed. A serious disadvantage in its use is the necessity of a second operation which carries the risk of increased morbidity.

Several synthetic materials have been used in the past, all of them having the same disadvantages: absent integration in the surrounding tissue, the long-term risk of infection resulting in extrusion of the inplant and the possible induction of malignancies (Huizing, 1974).

Therefore, nowadays preserved allogeneic rib cartilage is considered by most authors as the material of first choice, as it is easily available in sufficient quantity, easy to carve and shape and having a biomechanical behaviour comparable to septal cartilage.

However, there is still controversy about its long-term behaviour, particularly the incidence of resorption. This resorption is assumed to vary among grafts pre-treated by different methods.

Many investigations have been carried out to establish the most favourable method of preservation (Dingman and Grabb 1961, Kruger 1964, Krahl 1964, Hellmich 1972,1974, Eitschberger et al. 1978, Ersek et al. 1984). Attempts to improve the resistance to resorption by cobalt irradiation have not proven to be successfull (Hellmich 1972).

Recently, a technique was reported in which bovine cartilage was cross-linked by a combination of glutaraldehyde tanning and sterilising beta irradiation (Ersek et al. 1984). At present, the allogeneic rib cartilage is most commonly stored at 4 Celsius, immersed in merthiolate or Cialit (Huizing 1974, Hellmich 1974, McGlynn 1981). Maintaining the cartilage graft integrity remains an unresolved problem and operation results are quite often harmed by volume loss of the transplant.

Microwave irradiation is used in pathology laboratories for stabilization and subsequent preparation of tissue. Microwaves are electromagnetic waves which have a frequency between 300 MHz and 300 GHz. Industrial, scientific, and medical techologies employ several frequencies between 1GHz and 50 GHz. Domestic microwaves ovens, for instance, operate at 2.45 GHz. The first application of microwaves in pathology concerned fixation by internally generated heat (Mayers, 1970).

Application of microwaves in the field of reconstructive nasal surgery might have good prospects because first the microwave irradiation has effects on the proteins, probably influencing the acceptability of allogeneic cartilage as implant material, and second because microwave irradiation effects diffusion of reagents, such as alcohol, into tissue.

This study was designed to assess the effects of microwave irradiation on cartilage preservation.

Human rib cartilage immersed in Cialit or alcohol 70% was treated by different microwave dosages. The cartilage pieces were grafted to rabits, and specimens were compared in terms of histologic changes over a 10 months period.

## Material and methods

### Material

Rib cartilage was taken from fresh human cadavers, and cleaned from adhearing tissue, leaving the perichondrium intact, and stored in Cialit 1:5000 or alcohol 70%.

After storage during 14 days the cartilage was cut into pieces of 10x10 mm and 3 mm thickness.

Microwave irradiation was applied using a Miele M 696 with variation of immersion fluid, irradiation time, temperature and power level.

Pieces of cartilage were prepared as indicated in table 1.

### Animals

Eight adult rabbits weighing 1.5-1.9 Kg were sedated with Hypnorm in a dosis of 1 mg/kg i.m.

Incisions parallel to the vertebral column were made, and the various grafts were transplanted into the subcutaneous tissue of the back. The skin was sutered with Vicryl 4x0.

Postoperatively no antibiotic treatment was given. Cartilage implants were removed after 12 and 40 weeks.

Evaluation

The cartilage implants were prepared for histological examination.

In the histologic sections, the following aspects were evaluated: inflammatory infiltration, resorption and invasion of the cartilage by macrophages, and signs of necrosis. In every animal a ranking order from 1 to 7 was given for the microscopic appearence of the grafts.

Results

The irradiated cartilage kept its normal structure, firmness and elasticity and no difference could be observed macroscopically in comparisson to the untreated cartilage. Occasional dehiscence of the sutured skin edges occurred in 3 recipient places with subsequent inflammation and expulsion of the implant.

All other wounds healed well. The cartilage implants had become displaced over a small distance in several animals but could be easily identified. After 12 weeks of implantation the grafts nr. 7 (preserved in Cialit, not irradiated) could clearly be distinguished from the other grafts. Centrally in the cartilage necrosis with nuclear debris and granulocytes were present.

Numerous macrophages were concentrated around the edges of the graft, and extensive infiltration of macrophages into the cartilaginous tissue was present. The grafts treated with microwaves according the 6 other methods had clearly different microscopical aspects. In varying degree some invasion by macrophages and infiltration with lymphocytes and plasma cells around the graft was found. The differences between the microscopic appearance of the grafts irradiated according the various methods were slight, method 6 giving the poorest results and method 1 the best.

After 40 weeks of implantation the microscopic appearance of the microwaved grafts was clearly different. The untreated grafts again showed the most pronounced resorption by macrophages and centrally, the cartilage was fragmented with areas of necrotic tissue around the fragments. These changes were less severe than seen after 12 weeks of implantation. In the microwave treated series, some macrophages were seen around the edges and at some places invading the cartilage, but no fragmentation or central necrosis was observed. Progression of the resorption process, as seen in the untreated grafts could not be noticed. Also here, only slight differences between the methods were seen, again method 1 giving the best scores and method 6 the worst.

Discussion

Different treatments of the cartilage prior to implantation in order to modify the susceptibility of cartilage to resorption already were applied longer ago, but were never convincing enough. Recently, some other techniques were applied, such as decalcification with HCL (Pawlowski et al. 1982, Ksiazek et al. 1983) or processing with glutaraldehyde and beta irradiation (Ersek et al.1984). These techniques focus on denaturation of proteins or cross-linking of collagen molecules by which a probable decrease of antigenicity of the implant is achieved. The application of microwaves on cartilage may have comparable effects on the proteins and collagen fibers.

By microwaves, internally generated heat can be used for the denaturation of proteins and at higher temperatures the diffusion rate of reagents and chemical reaction rates become increased. Due to its homogeneous rise in temperature a denaturation of proteins and disulphide bond formation results in aggregation of proteins (Hopwood, 1984). By this, microwaved tissues contain probably a crude network of coagulated proteins through which the fixatives diffuse with more ease. Hence, the membranes of the cells are effected by the microwave treatment enhancing fixative diffusion (Boon en Kok, 1987). Cartilage matrix contain mainly collagen fibers and proteoglycan monomers, proteins to which chondroitin sulfate and keratan sulfate side chains are attached. Proteoglycan monomers are associated with a hyaluronic acid backbone forming large aggregates. The structural integrity of cartilage matrix is preserved by simultaneous interaction of keratan sulfate and chondroitin sulfate side chains of proteoglycan monomers to collagen fibers (Glant and Levai, 1983). Irradiation of cartilage with microwaves may alter these interactions either by the coagulative effects of the microwaves or the enhanced effects of the fixatives.

Our results in histological examination are comparable with those of other authors. Kruger (1964) found a reduction of the graft versus host reaction in case of cartilage preservation by mertiolate, formalin and alcohol. The formation of a surrounding capsule with granulation tissue invading and slowly absorbing the implant could not be completely prevented with these methods. In the experiments of Hellmich (1978) different preservation methods of human rib cartilage were compared. Implantation in rabbits showed cellular reaction with only few plasma cells and lymphocytes within the first 2 months of implantation and no cellular infiltration could be noticed after 6 months. Allogeneic implants gave the best results when preserved by mertiolate or lyophilisation. Histology on mertiolate preserved cartilage implanted in humans from 1 month to 14 years was done by Eitschberger et al. (1978). They found that the most resorption took place at the edges of the implants without increase of plasma cells and lymphocytes. The authors presumed the resorption as a result of a enzymatic process with infiltration of polymorphonuclear leucocytes and macrofages by which the chondromucoid gets dissolved and the collagen fibers dissociated. Ksiazek (1983) studied electronmicroscopically the mechanism of cartilage resorption in xenogeneic cartilage implantation and found macrophages to be the most important in the process of resorption. The abundant presence of lymphocytes might have influenced the macrophages by a release of lymfokines. Ersek et al. (1984) implanted bovine cartilage, processed by glutaraldehyde and beta radiation, in rabbits and found surface scalloping of the implants with moderate to dense infiltration of lymfocytes, eosinophiles, and histiocytes. The processed bovine cartilage appeared to behave as an inert foreign body with minimal resorption. It was supposed that the cartilage had become antigenically inert by the cross-linking effect of the processing.

McGlynn (1981) reported the use of Cialit for cartilage preservation. Histological examination of 2 specimen in place for up to 12 months showed complete absence of living chondrocytes with preservation of the normal cartilage matrix. The edges of the grafts were seen to be firmly fixed by fibrous tissue to the surrouding tissues.

The present pilot study indicates that microwave treatment of cartilage implants gives less resorption and less necrosis compared to untreated Cialit preserved cartilage.

It appears that microwave treatment in Cialit or alcohol 70% gives better results than microwave treatment without immersion fluid. Only slight differences existed between the 5 other microwave treatments. More experiments are needed in order to establish the optimal irradiation time, energy level, temperature and immersion fluid.

With the increasing incidence of acquired immunodeficiency syndrome (AIDS) and the consequent increasing risk of transmission of Human Immunodeficiency Virus (HIV), the use of human rib cartilage as allogeneic implant material may become discutable. Recently, inactivation of HIV by chemical desinfectants, such as alcohol (Spire et al. 1984) and also by heat treatment (56.C) was reported (Spire et al. 1985, Rouxioux et al. 1985, Houssein et al 1985, van der Meer et aL. 1986, Hancock et al. 1987).

Inactivation of HIV might even become the most important reason for additional microwave treatment of human rib cartilage for transplant purposes. If in addition alcohol is used as immersion fluid, killing of the virus might very well be achieved.

Literature

Boon M E, Kok L P Microwave Cookbook of Pathology. The art of Microscopic Visualization (1987) Coulomb Press Leyden, Leiden 1987

Dingman R O, Grabb W-C (1961) Costal cartilage homografts preserved by irradiation. Plast Reconstr Surg 28:562-567

Eitschberger E, Gammert C, Masing H, Pesch H J (1978) Zur Histologie und Resorption langzeit-implantierter Merthiolat-konservierter Knorpelspane in der Rhinoplastik. Laryngol Rhinol 57:440-444

Ersek R A, Greer D, Beisang A, Flynn P J, Denton D R (1984) Processed bovine cartilage: an improved biosynthetic implant for contour defects. Ann Plast Surg 12:317-408

Gibson T, Davis W B (1958) The long term survival of cartilage homografts in man. Br J Plast Surg 11:177-187

Giguere P (1982) Les homogreffes de cartilage costal irradie J Otolaryngol 11:379-384

Glant T, Levai G (1983) Localization of antigenic components in protecglycan aggregate of bovine nasal cartilage. Histochem 77:217-232

Hancock M R, Knapp M L, Ghany H C, Mayne P D (1987) Heat treatment for endocrinological investigations on plasma popsitive for human immunodeficiency vbirus (HIV). J Clin Pathol 40:409-411

Hellmich S (1972) Strahlenkonsevierung von Bankknorpel mit Kobalt-60. Arch Ohr-,Nas-u Kehlk,-Heilk 202:670-677 Hellmich S (1974) Der Einfluss unterschiedlicher Konservierungsmethoden auf die biologische Qualitat von Knorpelimplantaten. Laryngol Rhinol 53:711-717

Hopwood D Coghill G, Ramsay J, Milne G, Kerr M (1984) Microwave fixation: Its potential for routine techniques, histochemistry, immunochemistry, and electronmicroscopy. Histochem J 16:1171-1191

Houssein I, Wilcox H, Barron J (1985) Effect of heat treatment on results for bioc hemical analysis of plasma and serum. Clin Chem 31/12 2028-2030

Huizing E H (1974) Implantation and transplantation in reconstructive nasal surgery. Rhinology XII 93-106 Kruger E (1964) Absorption of human ribcartilage transplants to rabbits after preservation by different methods. Br J Plast Surg 17:254-263

Ksiazek T, Thyberg J (1983) Electrinmicroscopic studies on 7 resorption of xenogeneic cartilage implants. Virchows Arch (Pathol Anat) 399:79-88

Mayers C P (1970) Histological fixation by microwave heating J Clin Pathol 23:273-275

McGlynn J, Sharpe D T (1981) Cialit preserved homograft cartilage in nasal augmentation. A long term review. Br J Plast Surg 34:53-57

van der Meer J, Daenen s, van Imhoof G W, de Wolf J T M, Halie M R, (1986) Absence of seroconversion for HLTV-III in haemophiliacs intensively treated with heat treated factor VIII concentrate. Br Med J 292:1049

Pawlowski A, Malejczyk J, Ksiazek T, Moskalewski S (1982) Resorption of calcified and decalcified costal cartilage. Chir Plastica 7:59-66

Reck R, Mika H, Sonntag W (1979) Allogeneic implants of the nasal dorsum: clinical and experimental studies in animals. Rhinology XVII 121-124

Schuller D E, Bardach J, Krause C J (1977) Irradiated homologous costal cartilage for facial contour restauration. Arch Otolaryngol 103:12-17

Spire B, Barre-Sinoussi F, Montagnier L, et al. (1984) Inactivation of lymphadenopathy associated virus by chemical desinfectants. Lancet ii:899-901

Spire B, Dormont D, Barre-Sinoussi F, Montagnier L, Chermann J C, (1985) Inactivation of lymphadenopathy associated virus by heat, gamma rays, and ultraviolet light. Lancet i:188-189

Table 1

| Various microwave treatments | | | | | | |
|---|---|---|---|---|---|---|
| 1. | Cialit, | irradiation | 5 min. | temp. | 50oC, | 150 Watt. |
| 2. | Cialit, | irradiation | 20 min. | temp. | 50oC, | 150 Watt. |
| 3. | Cialit, | irradiation | 10 min. | temp. | 80oC, | 450 Watt. |
| 4. | Alc. 70%, | irradiation | 5 min. | temp. | 60oC, | 150 Watt. |
| 5. | Alc. 70%, | irradiation | 10 min. | temp. | 60oC, | 150 Watt. |
| 6. | dry, | irradiation | 3 min. | temp. | 60oC, | 80 Watt. |
| 7. | Cialit, | no irradiation | | | | |

Table 2

| Ranking order histological examination | | |
|---|---|---|
| treatment nr. | ranking order after 12 weeks | ranking order after 40 weeks |
| 1 | 1 | 1 |
| 2 | 2 | 3 |
| 3 | 5 | 5 |
| 4 | 4 | 4 |
| 5 | 3 | 2 |
| 6 | 6 | 6 |
| 7 | 7 | 7 |

DIA TEKST-DIA ZWART


DIA NEUS-DIA NEUS

Resorption of allogeneic cartilage implants is one of the main problems in corrective facial and nasal surgery. Maintaining the cartilage graft integrity remains an unresolved problem and results of operation like this are quite often harmed by volume loss of the transplant.

However, the use of preserved costal cartilage has still proven to be indispensible and nowadays preserved allogeneic rib cartilage is considered by most of our colleagues as the material of first choice, as it is easily available in sufficient quantity, easy to carve and shape and having a biomechanical behaviour comparable to septal cartilage.

However, there still is a controversy about its long-term behaviour, particularly the incidence of resorption. This resorption is assumed to vary among grafts pre-treated by different methods.


DIA KIP-DIA TEKST

Microwaves are electromagnetic waves which have a frequency between 300 MHz and 300 GHz.,corresponding with wave lenghts between 1 m and 1mm respectively.
Microwave ovens provide an effective way of heating many nonconductive materials. The microwaves penetrate the material which is heated uniformly throughout the body.


DIA OVEN-DIA TEKST

In pathology laboratories microwave irradiation is used for stabilization and fixation of tissue by internally generated heat.

We thougt that in the field of reconstructive nasal surgery application of microwaves might have good prospects in preserving cartilage, because the microwave irradiation has effects on the proteins, and because microwave irradiation effects diffusion of reagents, such as alcohol or Cialit into tissue.


DIA LIJSTJE-DIA ZWART

In order to assess the effects of microwave irradiation on cartilage preservation, microwave irradiation was applicated on human rib cartilage with variation of immersion fluid, irradiation time, temperature and power level.

Pieces of this cartilage were grafted subcutaneously in the back of rabbits, and removed after 12 and 40 weeks. The specimens were prepared for histological examination and compared in terms of histologic changes.


Results

The irradiated cartilage kept its normal structure, firmness and elasticity and no difference could be observed in comparison to the untreated cartilage.


DIA OVERZICHT -DIA OVERZICHT

Here you see the results after 12 weeks of implantation. The non-irradiated grafts preserved in Cialit, can clearly be distinguished from the other grafts.


DIA -DIA

Centrally in the cartilage necrosis with nuclear debris and granulocytes is present. There is a dense infiltration around the edges of the graft, and in the graft you see dead chondrocytes.

The grafts treated with microwaves according the 6 other methods presented clearly different microscopical aspects. A moderate cellular infiltration around the graft was found in varying degree, but no signs of necrosis.

DIA OVERZICHT-DIA OVERZICHT

You must disregard the color differences which are caused by the Papanicoulao color technique, they are without significance.

After 40 weeks of implantation the microscopic appearance of the microwaved grafts differed again clearly from the untreated grafts.

DIA-DIA

The untreated grafts showed the most pronounced destruction by macrophages. In the center, the cartilage was fragmented with large areas of necrotic tissue around the fragments. In the microwave treated series, some macrophages were seen around the edges and at some places invading the cartilage, but no fragmentation or central necrosis was observed. Progression of the resorption process, as seen in the untreated grafts, could not be noticed. The differences in the microscopic appearance of the grafts irradiated according the various methods were slight.

DIA ZWART -DIA ZWART

Discussion

Cartilage consists of a network of collagen fibers in a groundsubstance containing mainly proteoglycan monomers, The structural integrity of cartilage matrix is preserved by the interaction of these proteins with collagen fibers.

DIA TEKST-DIA ZWART

By microwaves, internally generated heat can be used for the denaturation of proteins and at higher temperatures the diffusion rate of reagents and chemical reaction rates increases.

Due to its homogeneous rise in temperature a denaturation of proteins and disulphide bond formation results in aggregation of proteins. By this, microwaved tissues contain probably a crude network of coagulated proteins through which the fixatives diffuse more easily.

Irradiation of cartilage with microwaves may alter these interactions either by the coagulative effects of the microwaves or the enhanced effects of the fixatives.

IN CONCLUSION MR. CHAIRMAN, The present pilot study indicates that additional microwave treatment of cartilage implants seems to give less resorption and less necrosis compared to cartilage preserved in Cialit only.

DIA TEKST-DIA ZWART

The best results were achieved when alcohol was used as immersionfluid and with an irradiation time of 5 or 10 minutes at 60 degrees Celsius. Using Cialit gave slightly less results and poor results were obtained when no immersion fluid was used. We now have only compared the use of Cialit with and without irradiation, but in our latest experiments especially the use of glutaraldehyde in combination with microwave irradiation seems promising.

LAST SLIDES PLEASE

I want to make a final remark about another aspect of microwave irradiation. With the increasing incidence of the acquired immunodeficiency syndrome, use of human rib cartilage as allogeneic implant material may become disputable. Recently, inactivation of the AIDS virus by chemical desinfectants, such as alcohol and also by heat treatment (56.C) was reported. Inactivation of this virus might even become the most important reason for additional microwave treatment of human rib cartilage.

If, in addition, alcohol or glutaraldehyde is used as immersion fluid, killing of the virus might very well be achieved.

## Claims

A method of preservation of bone, especially human cartilage, in which a bone structure is exposed to microwave irradiation.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-8 500 954 (THE TRUSTEES OF COLUMBIA UNIVERSITY IN NEW YORK) <br> * Claim 11 * <br> --- | | A 01 N 1/02 |
| A | US-A-4 456 589 (D.G. HOLMAN et al.) <br> * Column 2, lines 10,11; claims 1,2 * <br> --- | | |
| A | FR-A-1 317 584 (OLIN MATHIESON) <br> * Abstract * <br> --- | | |
| A | EP-A-0 015 055 (COLLAGEN CORP.) <br> * Page 3, lines 10,19 * <br> ----- | | |

TECHNICAL FIELDS
SEARCHED (Int. Cl.4)

A 01 N 1/02
A 61 L 27/00

The present search report has been drawn up for all claims

| Place of search | Date of completion of the search | Examiner |
|---|---|---|
| THE HAGUE | 26-01-1989 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................

& : member of the same patent family, corresponding document

EPO FORM 1503 03.82 (P0401)